# EUROPEAN PATENT APPLICATION

(11) **EP 3 051 132 A1**
(43) Date of publication of application: **03.08.2016**
(21) Application number: 16151251.2
(22) Date of filing: 14.01.2016
(51) Int. Cl.: F04B 43/12, F04B 49/06, F04B 49/20

(54) **LIQUID TRANSPORTING APPARATUS AND LIQUID TRANSPORTING METHOD**

(30) Priority: 16.01.2015 JP 2015006507
(71) Applicant: Seiko Epson Corporation, Tokyo 163 (JP)
(72) Inventor: Miyamoto, Tsutomu, Nagano, 392-8502 (JP)
(74) Representative: Hoffmann Eitle

(57) **Abstract**

A liquid transporting apparatus includes a pump unit that transports a liquid, a battery housing unit that houses a battery supplying power for driving the pump unit; a driving unit that drives the pump, a control unit that controls the driving unit, and a temperature measuring unit that measures a temperature. The control unit controls driving of the pump unit by a first mode in which the pump unit is driven by a predetermined driving amount in unit time and a second mode in which the pump is driven by a driving amount that is smaller than the predetermined driving amount in unit time that is shorter than the unit time described above when the temperature measured by the temperature measuring unit is lower than a predetermined value.

## Description

### BACKGROUND

### 1. Technical Field

The present invention relates to a liquid transporting apparatus and a liquid transporting method.

### 2. Related Art

As a liquid transporting apparatus transporting a liquid, an apparatus disclosed in JP-A-2013-24185 has been known. In the apparatus disclosed in JP-A-2013-24185, a plurality of fingers are disposed along a tube, a cam sequentially presses a tube, the tube is squeezed, and then the liquid is transported. Furthermore, in the apparatus disclosed in JP-A-2013-24185, since power consumption is large if driving and stopping are repeated little by little, a pump is driven by a driving amount greater than a predetermined driving amount for unit time longer than as normal when a remaining quantity of a battery is low.

Power (current) supplied from a battery is decreased at a low temperature. If a pump is driven as normal under circumstances that supply power from the battery is reduced, there is a concern that a voltage that is required in a circuit within the apparatus cannot be ensured and an operation of the apparatus is unstable.

### SUMMARY

An advantage of some aspects of the invention is to stably operate an apparatus even during a low temperature.

An aspect of the invention is directed to a liquid transporting apparatus including a pump that transports a liquid; a battery housing unit that houses a battery supplying power for driving the pump; a control unit that controls driving of the pump; and a temperature measuring unit that measures a temperature. The control unit controls driving of the pump by a first mode in which the pump is driven by a predetermined driving amount per unit time and a second mode in which the pump is driven by a driving amount that is smaller than the predetermined driving amount in unit time that is shorter than the unit time described above when the temperature measured by the temperature measuring unit is lower than a predetermined value.

The other features of the invention will be apparent from the present specification and accompanying drawings.

### BRIEF DESCRIPTION OF THE DRAWINGS

The invention will be described with reference to the accompanying drawings, wherein like numbers reference like elements.
Fig. 1 is a block diagram of a liquid transporting apparatus of a first embodiment.
Fig. 2A is a plan view of a pump viewed from a direction parallel to a rotary shaft of a cam.
Fig. 2B is a sectional view that is taken along line IIB-IIB of Fig. 2A.
Fig. 3A is an explanatory view of an output current of a boosting circuit (DC-DC converter).
Fig. 3B is an explanatory view of a waveform of a battery voltage during normal.
Fig. 3C is an explanatory view of a waveform of the battery voltage during a low temperature.
Fig. 4A is an explanatory view of an output current of the boosting circuit (DC-DC converter) during a normal mode.
Fig. 4B is an explanatory view of an output current of the boosting circuit (DC-DC converter) during a low temperature mode.
Figs. 5A and 5B are conceptual explanatory views of a drop amount of the battery voltage in the normal mode and the low temperature mode.
Fig. 6A is an explanatory view of an output current of a boosting circuit (DC-DC converter) during a normal mode of a second embodiment.
Fig. 6B is an explanatory view of an output current of the boosting circuit (DC-DC converter) during a low temperature mode of the second embodiment.
Fig. 6C is an explanatory view of an output current of the boosting circuit (DC-DC converter) during an extremely low temperature mode of the second embodiment.
Fig. 7 is a graph of a relationship between a rotation amount of a cam and an accumulative transport amount.
Fig. 8A is an explanatory view of backflow of a liquid in a backflow period.
Fig. 8B is an explanatory view of backflow of the liquid in the backflow period.
Fig. 9 is a block diagram of a liquid transporting apparatus of a fifth embodiment.

### DESCRIPTION OF EXEMPLARY EMBODIMENTS

At least the following matters will be made clear by the description of this specification and the accompanying drawings.

A the liquid transporting apparatus will become apparent which includes a pump that transports a liquid; a battery housing unit that houses a battery supplying power for driving the pump; a control unit that controls driving of the pump; and a temperature measuring unit that measures a temperature. The control unit controls driving of the pump by a first mode in which the pump is driven by a predetermined driving amount per unit time and a second mode in which the pump is driven by a driving amount that is smaller than the predetermined driving amount in unit time that is shorter than the unit time described above when the temperature measured by the temperature measuring unit is lower than a predetermined value. According to such a liquid transporting apparatus, it is possible to stably operate the apparatus even during the low temperature.

It is preferable that a boosting circuit boosting a voltage of the battery is provided and power is supplied to the pump through the boosting circuit. Thus, it is possible to stabilize output power of the boosting circuit.

It is preferable that other power consumption elements separate from the pump is further provided and the power consumption element can be driven independently from the control unit. It is particularly effective when the control unit cannot control power consumption timing of power consumption element.

The pump transports the liquid by squeezing the tube by sequentially pressing a plurality of fingers disposed along the tube by the cam. It is preferable that in the first mode, the control unit performs a skip drive for driving the cam so as to skip a period in which the liquid is not transported by closing a tube by a finger of the most downstream side of the plurality of fingers and a period in which the liquid is flows back by opening the finger of the most downstream side, and in the second mode, the control unit does not perform the skip drive. Thus, it is possible to transport the liquid with high accuracy during normal and to stably operate the apparatus during the low temperature.

It is preferable that the battery housing unit has a heat insulating material for insulating the battery. Thus, it is possible to suppress a decrease in a temperature of the battery.

It is preferable that the temperature measuring unit is housed in the battery housing unit together with the battery. Thus, it is possible to accurately measure the temperature of the battery.

A liquid transporting method for transporting a liquid by driving a pump by power of a battery supplied from the battery will become apparent, which includes driving a pump by a predetermined driving amount by unit time, and driving the pump by a driving amount that is smaller than the predetermined driving amount in unit time shorter than unit time described above when the temperature measured by the temperature measuring unit is lower than a predetermined value. According to such a liquid transporting method, it is possible to stably operate the apparatus even during the low temperature.

### First Embodiment

### Configuration of Liquid Transporting Apparatus 1

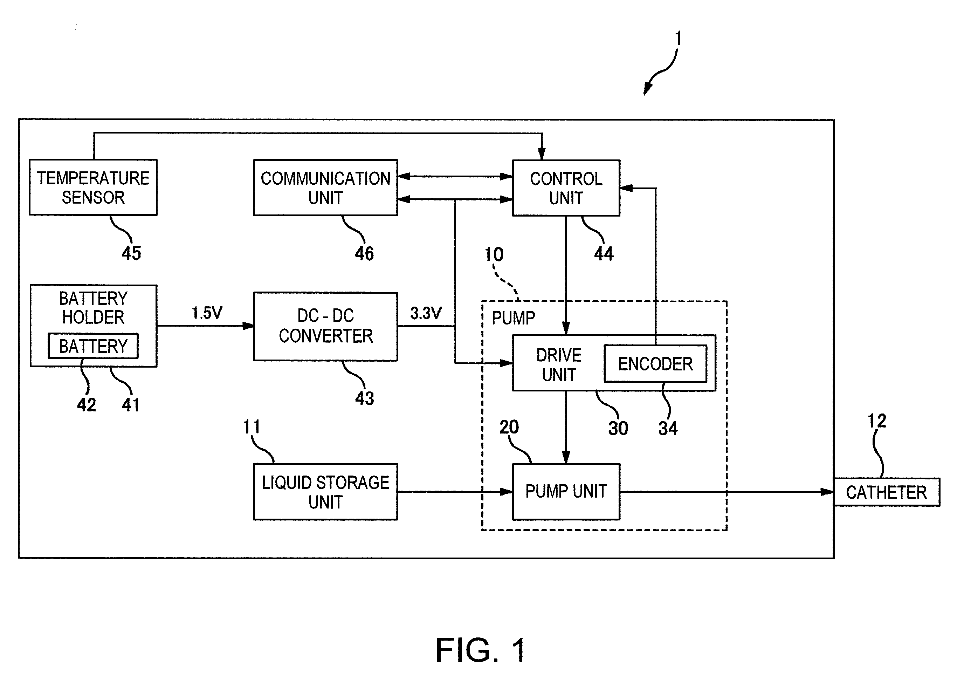

Fig. 1 is a block diagram of the liquid transporting apparatus 1 of the first embodiment. The liquid transporting apparatus 1 is an apparatus for transporting a liquid by driving a pump 10. Here, the liquid transporting apparatus 1 is an insulin pump and the pump 10 supplies insulin (medical solution) that is stored in a liquid storage unit 11 to a catheter 12, and injects the medical solution into a living body from the catheter 12.

The liquid transporting apparatus 1 has the pump 10, a battery housing unit 41, a boosting circuit (DC-DC converter) 43, a control unit 44, a temperature measuring unit (temperature sensor) 45, a communication unit 46. A battery 42 is housed in the battery housing unit 41 and a power supply voltage of 1.5 V is supplied from the battery 42. The battery 42 is detachably mounted and when the liquid transporting apparatus 1 is shipped from a factory, the liquid transporting apparatus 1 does not include the battery 42. Similarly, the liquid storage unit 11 may be a detachable cartridge type.

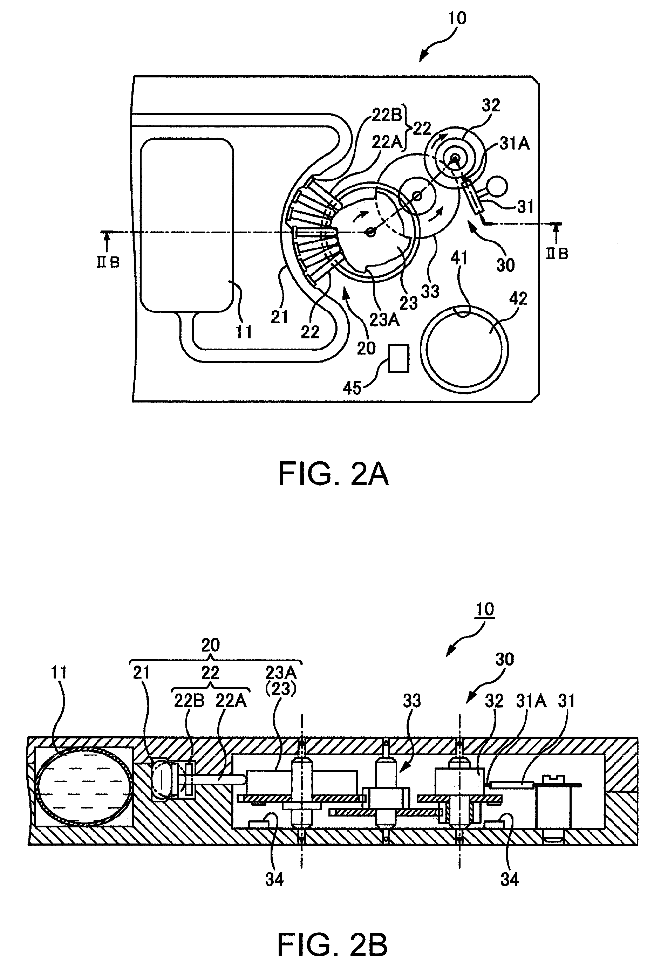

Figs. 2A and 2B are explanatory views of a configuration of the pump 10. Fig. 2A is a plan view of the pump 10 viewed in a direction parallel to a rotary shaft of a cam 23. Fig. 2B is a sectional view of a line IIB-IIB of Fig. 2A.

The pump 10 is a device for transporting the liquid stored in the liquid storage unit 11. The pump 10 has a pump unit 20 and a drive unit 30.

The pump unit 20 includes a tube 21, a plurality of fingers 22, and the cam 23.

The tube 21 is a pipe for transporting the liquid. An end of the tube 21 communicates with the liquid storage unit 11 and the other end communicates with the catheter 12. The tube 21 has elasticity to an extent to be closed when the tube 21 is pressed by the fingers 22 and to return to an original when a force from the fingers 22 is released. The tube 21 is disposed in a partially circular shape. The portion having a partially circular shape is disposed between a wall portion and the fingers 22, and the rotary shaft of the cam 23 is disposed in an arc center.

The finger 22 is a member for closing the tube 21. The finger 22 is driven to be operated by receiving a force from the cam 23. The finger 22 has a rod-shaped shaft unit 22A and a flange-shaped pressing unit 22B, and has a T-shape. The finger 22 is supported such that the shaft unit 22A is movable in an axial direction. One end of the shaft unit 22A on the cam 23 side comes into contact with the cam 23 and the pressing unit 22B disposed in the other end of the shaft unit 22A comes into contact with the tube 21.

The plurality of fingers 22 are disposed along the tube 21 and are radially disposed from a rotation center of the cam 23. The plurality of fingers 22 are disposed between an outer surface of the cam 23 and the tube 21. Here, seven fingers 22 are provided and it may be referred as a first finger 22-1, a second finger 22-2, ···, and a seventh finger 22-7 in order from an upstream side in a transport direction of the liquid.

The cam 23 is a member for sequentially pressing the plurality of fingers 22 by being rotated. The cam 23 sequentially presses the plurality of fingers 22 and thereby the tube 21 is squeezed and the liquid is transported. A plurality (here, four) of protrusion units 23A for pressing the fingers 22 are formed on an outer periphery of the cam 23. When the fingers 22 are disengaged from the protrusion unit 23A, the tube 21 returns to the original shape by an elastic force of the tube 21. In order to prevent backflow of the liquid, the plurality of protrusion units 23A are formed such that at least one of the fingers 22 closes the tube 21.

The drive unit 30 drives the cam 23 to be rotated. The drive unit 30 includes a piezoelectric actuator 31, a rotor 32, a deceleration transmission mechanism 33.

The piezoelectric actuator 31 is a member for rotating the rotor 32 by using vibration of a piezoelectric element. The piezoelectric actuator 31 includes the piezoelectric element (not illustrated) and a vibrating body having a contact portion 31A in an end projected. If the piezoelectric element is vibrated, the vibrating body vibrates so that the contact portion 31A draws an elliptical orbit. Moreover, the contact portion 31A is vibrated so as to drawing the elliptical orbit by exciting a longitudinal primary vibration mode in which the rectangular vibrating body expands and contracts in a longitudinal direction and a bending secondary vibration mode in which the vibrating body is bent in the direction orthogonal to the longitudinal direction to the vibrating body. Thus, the vibrating body has a shape having a natural frequency so as to excite the longitudinal primary vibration mode and the bending secondary vibration mode. The contact portion 31A comes into contact with the rotor 32 and if the contact portion 31A vibrates while drawing the elliptical orbit, the rotor 32 is driven to be rotated.

The rotor 32 is a driven body that is rotated by the piezoelectric actuator 31. The deceleration transmission mechanism 33 is a mechanism (reduction gear) for transmitting the rotation of the rotor 32 to the cam 23 at a predetermined reduction ratio.

As illustrated in Fig. 1, the drive unit 30 has an encoder 34. The encoder 34 is a detection unit for detecting a rotation amount of the cam 23. Here, an encoder 34 is a rotary encoder including a light emitting unit and a light receiving unit. The encoder 34 irradiates the rotor 32 with a detection light from the light emitting unit, detects a change in the detection light (reflected light or transmitted light) by marks or slits formed on the rotor 32 by the light receiving unit, and detects a count value (rotation amount of the rotor 32) according to a detection result of the light receiving unit. Since the reduction ratio of the deceleration transmission mechanism 33 is known, it is possible to detect the rotation amount of the cam 23 from the rotation amount of the rotor 32. However, the encoder 34 may directly detect the rotation amount of the cam 23. Moreover, if the encoder 34 includes an original point sensor, since the rotation amount can be detected from an original point (reference position) of the cam 23, it is also possible to detect a rotation position of the cam 23. The encoder 34 outputs the count value (the rotation amount of the rotor 32) to the control unit 44.

The battery housing unit 41 is a housing unit (battery housing unit) for holding the battery 42. As described above, the battery housing unit 41 detachably houses the battery 42. Here, the battery 42 is a flat battery such as a button-type battery or a coin-type battery having relatively low battery capacity. The battery 42 housed in the battery housing unit 41 outputs power of 1. 5 V to the boosting circuit (DC-DC converter) 43.

The boosting circuit (DC-DC converter) 43 is a boosting circuit for boosting a voltage of the battery 42. Here, the boosting circuit (DC-DC converter) 43 boosts the power supply voltage (battery voltage) of 1.5 V to 3.3 V. The boosting circuit (DC-DC converter) 43 supplies power to the drive unit 30 (piezoelectric actuator 31 and the like) of the pump 10. Furthermore, the boosting circuit (DC-DC converter) 43 also supplies power to other power consumption elements separate from the drive unit 30 of the pump 10. Specifically, the boosting circuit (DC-DC converter) 43 also supplies power to the communication unit 46, the control unit 44, a clock circuit (not illustrated), a memory, and the like.

The control unit 44 is a controller performing a control of the liquid transporting apparatus 1. As described below, the control unit 44 can drive the pump 10 in predetermined drive modes (normal mode and low temperature mode) by outputting predetermined drive signals to the drive unit 30.

The temperature measuring unit (temperature sensor) 45 outputs a measured result to the control unit 44. As described below, the control unit 44 switches the drive modes of the pump 10 (normal mode and low temperature mode) based on the measured result of the temperature measuring unit (temperature sensor) 45.

The communication unit 46 is a communication circuit for transmitting and receiving information to and from an external device. For example, the communication unit 46 is a communication circuit in compliance with a short distance wireless communication standard such as Bluetooth (registered trademark). Moreover, when using a generic device (generic chip) as the communication unit 46, the communication unit 46 can be driven independently from the control unit 44 and performs communication at predetermined timing even without a command from the control unit 44.

### Liquid Transporting Method

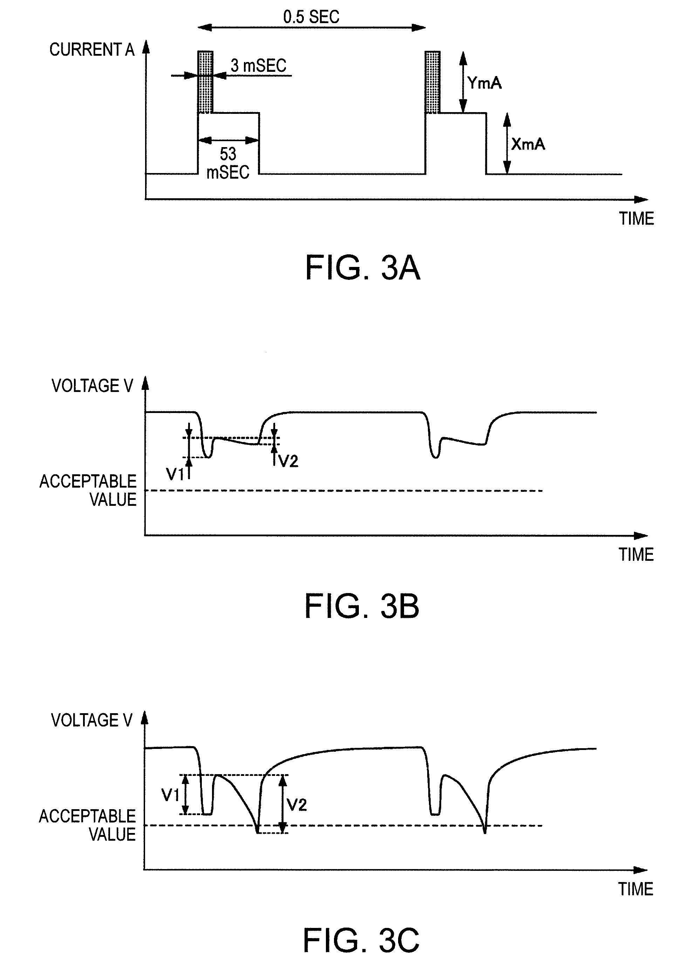

Fig. 3A is an explanatory view of an output current of the boosting circuit (DC-DC converter) 43 (however, for the sake of convenience of description, a length of a horizontal axis of a graph (time axis) in the view does not fully coincide with a length of time). The boosting circuit (DC-DC converter) 43 supplies power to the pump 10 and the communication unit 46. In the graph in the view, shading is applied to portions corresponding to the current supplied to the communication unit 46. When driving the pump 10, a current of X milliamps is supplied from the boosting circuit (DC-DC converter) 43 to the pump 10. Furthermore, a current of Y milliamps is supplied from the boosting circuit (DC-DC converter) 43 to the communication unit 46 during communication of the communication unit 46. Moreover, here, an output voltage of the boosting circuit (DC-DC converter) 43 is 3.3 V.

A time change in the output current of the boosting circuit (DC-DC converter) 43 is illustrated in the view when driving the pump 10 in the normal mode. The normal mode is a transport system for transporting the liquid by driving the pump 10 by a predetermined driving amount per unit time. Here, the control unit 44 transports the liquid by driving the pump 10 such that the unit time (repetition period) is 0.5 seconds and the encoder 34 detects the rotation amount of the rotor 32 of 20 counts in unit time. As illustrated in the view, the control unit 44 does not continuously rotate the rotor 32 during the unit time (0.5 seconds) at a constant speed, but drives the pump 10 until the encoder 34 detects the rotation amount of 20 counts (rotates the rotor 32), and then temporarily stops the drive of the pump 10 to a start of the next unit time. This is because an elliptical vibration cycle of the contact portion 31A of the piezoelectric actuator 31 is determined by a natural frequency of a vibration plate and the elliptical vibration cycle of the contact portion 31A of the piezoelectric actuator 31 cannot be arbitrarily changed.

When driving the pump 10, a drive current of a predetermined current amount (X milliamps) is supplied from the boosting circuit (DC-DC converter) 43 to the pump 10. Furthermore, here, the time until the encoder 34 detects the rotation amount of 20 counts is approximately 53 milliseconds. That is, it is repeated every 0.5 seconds that the current of X milliamps (3.3 V) flows from the boosting circuit (DC-DC converter) 43 to the pump 10 by approximately 53 milliseconds.

The drive current of a predetermined current amount (Y milliamps) is supplied from the boosting circuit (DC-DC converter) 43 to the communication unit 46 during communication of the communication unit 46. Here, the current is supplied from the boosting circuit (DC-DC converter) 43 to the communication unit 46 by 3 milliseconds during communication.

If the generic device (generic chip) is used as the communication unit 46, even though a command does not come out from the control unit 44, the communication unit 46 can perform communication at a predetermined timing. That is, the control unit 44 is in a state of unable to control power consumption timing of the communication unit 46. Here, the communication unit 46 performs communication of 3 milliseconds at the same timing as the start of driving of the pump 10. Moreover, although the communication unit 46 performs communication at other timing, since power consumption timing of the communication unit 46 cannot be controlled by the control unit 44, here, it is assumed worst timing. The output current of the boosting circuit (DC-DC converter) 43 during communication at least becomes X+Y milliamps.

Fig. 3B is an explanatory view of a waveform of the battery voltage during normal. Fig. 3C is an explanatory view of a waveform of the battery voltage during the low temperature. Moreover, the battery voltage is a voltage on an input side of the boosting circuit (DC-DC converter) 43. Dot lines in the views indicate a voltage value (acceptable value) required for maintaining the output voltage of 3.3 V by the boosting circuit (DC-DC converter) 43.

As illustrated in Figs. 3B and 3C, the battery voltage is decreased when continuously outputting the drive current of the predetermined current amount (X milliamps) from the boosting circuit (DC-DC converter) 43 to the pump 10 when driving the pump 10. Furthermore, as illustrated in Fig. 3C, the decrease in the battery voltage is remarkable when driving the pump 10 during the low temperature. This is because a current supply ability of the battery 42 during the low temperature is lowered more than that during normal.

Furthermore, since the boosting circuit (DC-DC converter) 43 outputs relatively large current (at least X+Y milliamps) during communication, the voltage on the input side of the boosting circuit (DC-DC converter) 43 is greatly decreased. Since the output current of the boosting circuit (DC-DC converter) 43 is reduced after the communication completion, the battery voltage is slightly recovered. However, even after the battery voltage is slightly recovered after communication completion, since driving of the pump 10 is continued, the battery voltage is decreased during driving the pump 10 even after the communication completion.

As illustrated in Fig. 3C, since the drop of the battery voltage is remarkable when driving the pump 10 during the low temperature, a drop amount V2 of the battery voltage by driving the pump 10 after the communication completion is greater than a recovery amount V1 of the battery voltage after the communication completion. As a result, even if the battery voltage does not fall to the acceptable value or less when the communication timing of the communication unit 46 and the driving timing of the pump 10 are overlapped during the low temperature (in other words, even if the battery voltage is the acceptable value or less when the boosting circuit (DC-DC converter) 43 outputs the X+Y milliamps for 3 milliseconds), there is a concern that the battery voltage is the acceptable value or less after the communication completion in the low temperature. If the battery voltage is the acceptable value or less, the output voltage (3.3 V) of the boosting circuit (DC-DC converter) 43 is dropped and, as a result, there is a concern to lead to malfunction of a logic circuit of the control unit 44 and the like.

As illustrated in Figs. 3B and 3C, the drop of the battery voltage during driving of the pump 10 becomes large as a time for continuing the driving of the pump 10 (continuous driving time, here, approximately 53 milliseconds) is long. Thus, if the continuous driving time of the pump 10 is reduced, it is possible to suppress the drop of the battery voltage. Thus, when a temperature measured by the temperature measuring unit (temperature sensor) 45 is lower than a predetermined value, the control unit 44 performs the low temperature mode as described below.

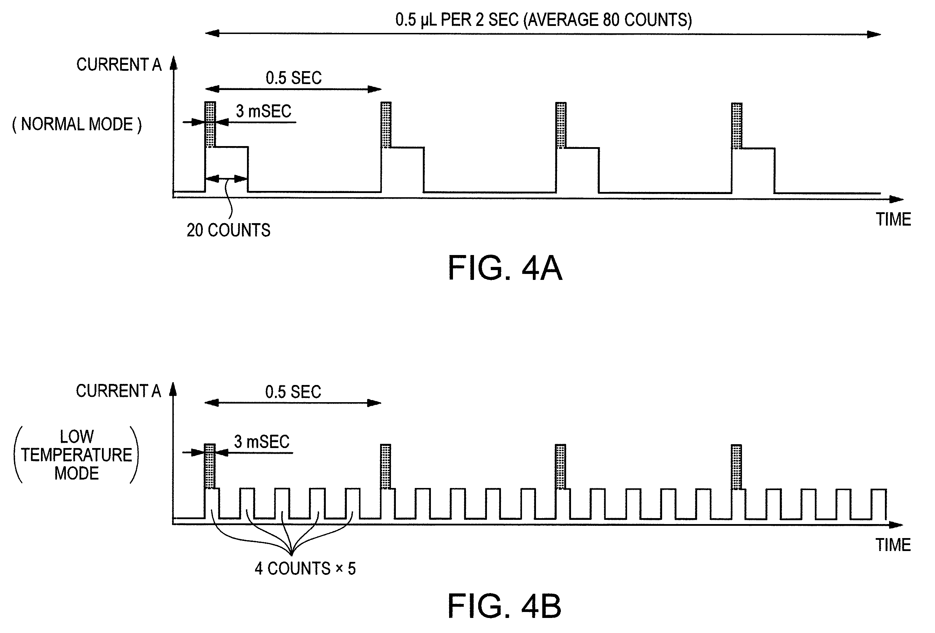

Fig. 4B is an explanatory view of the output current of the boosting circuit (DC-DC converter) 43 during the low temperature mode. In the graph in the view, shading is applied to portions corresponding to the current supplied to the communication unit 46. Moreover, for comparison, an explanatory view indicating the output current of the boosting circuit (DC-DC converter) 43 during the normal mode is illustrated in Fig. 4A. Since the output current during the normal mode illustrated in Fig. 4A is the same as the output current illustrated in Fig. 3A, description will be omitted.

The low temperature mode is a transport system for transporting the liquid by driving the pump 10 by a driving amount that is smaller than the driving amount (here, 20 counts) during the normal mode in the unit time that is shorter than the unit time (here, 0.5 seconds) during the normal mode when the temperature measured by the temperature measuring unit (temperature sensor) is lower than a predetermined value. Specifically, the control unit 44 transports the liquid by driving the pump 10 such that the unit time (repetition period) is 0.1 second and the encoder 34 detects the rotation amount of the rotor 32 of 4 counts in unit time when the temperature measured by the temperature measuring unit (temperature sensor) 45 is lower than 10°C during the low temperature mode. The control unit 44 does not continuously rotate the rotor 32 during the unit time (0.1 second in the low temperature mode) at a constant speed, but drives (rotates the rotor 32) the pump 10 until the encoder 34 detects the rotation amount of 4 counts, and then temporarily stops the drive of the pump 10 to a start of the next unit time.

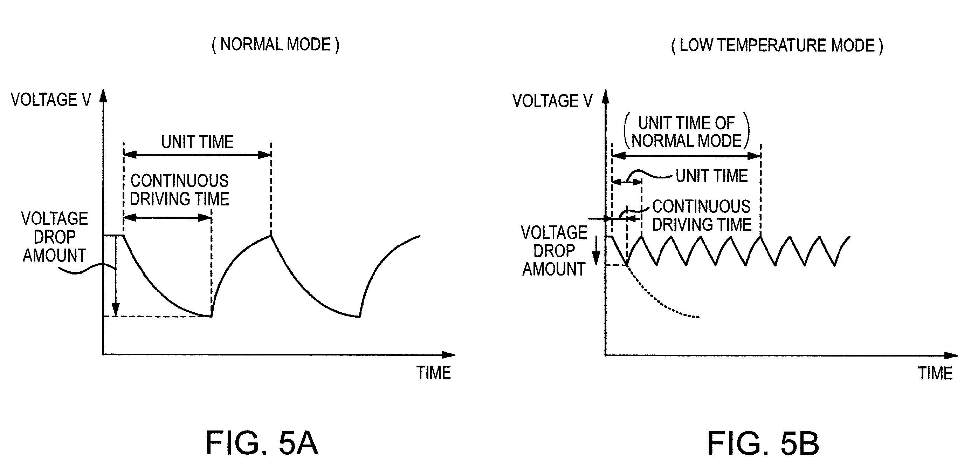

Figs. 5A and 5B are conceptual explanatory views of a drop amount of the battery voltage in the normal mode and the low temperature mode. As indicated by a graph on the left side in the view, if the pump 10 is driven in the normal mode, duration (continuous driving time) of drive of the pump 10 is long and, as a result, a voltage drop amount is great. On the other hand, as indicated by a graph on the right side in the view, if the pump 10 is driven in the low temperature mode, the continuous driving time of drive of the pump 10 can be reduced. Specifically, in the low temperature mode, since the control unit 44 drives the pump 10 by the driving amount of 4 counts, it is possible to reduce the continuous driving time of the pump 10 compared to the normal mode in which the pump 10 is driven by the driving amount of 20 counts. Thus, if the pump 10 is driven in the low temperature mode, it is possible to suppress the drop of the battery voltage during driving of the pump 10 even if during the low temperature in which the current supply ability of the battery 42 is decreased.

Moreover, in the low temperature mode, the control unit 44 drives the pump 10 in the same driving amount (20 cycles) as the normal mode in the unit time during the normal mode by repeating drive and stop of the pump 10 5 times in the unit time (0.5 seconds) during the normal mode. That is, in the low temperature mode, the control unit 44 drives the pump 10 by dividing the driving amount (20 counts) of 1 time during the normal mode into a plurality of times. Thus, in the low temperature mode, the driving amount per 1 time driving is 4 counts and is smaller than the driving amount (20 counts) during the normal mode, but average driving amounts per unit time are substantially same as each other.

During communication of the communication unit 46, similar to during the normal mode, the drive current of the predetermined current amount (Y milliamps) is supplied from the boosting circuit (DC-DC converter) 43 to the communication unit 46 by 3 milliseconds. Then, if the communication timing of the communication unit 46 overlaps with the driving timing of the pump 10, the boosting circuit (DC-DC converter) 43 outputs a relatively large current similar to the normal mode. However, in the low temperature mode, since the driving time of the pump 10 is short after the communication completion compared to the normal mode, the drop amount (corresponding to V2 of Fig. 3C) of the battery voltage after the communication completion is decreased. Thus, in the low temperature mode, the recovery amount (corresponding to V1 of Fig. 3C) of the battery voltage after the communication completion is likely to be greater than the drop amount (corresponding to V2 of Fig. 3C) of the battery voltage by driving of the pump 10 after the communication completion. Thus, if a design is performed such that the battery voltage does not become the acceptable value or less when the communication timing of the communication unit 46 and the driving timing of the pump 10 are overlapped, it is possible to suppress that the battery voltage becomes the acceptable value or less by driving the pump 10 after the communication completion by performing the low temperature mode. As a result, the output voltage (3.3 V) of the boosting circuit (DC-DC converter) 43 is likely to be maintained and it is possible to suppress malfunction of the logic circuit of the control unit 44 and the like.

Moreover, since efficiency of the low temperature mode is poor compared to the normal mode, the control unit 44 does not perform the low temperature mode, but performs the normal mode if it is not the low temperature. The reason the efficiency of the low temperature mode is poor, is acceleration of the rotor 32 (making the rotor 32 from a stopped state to a rotating state) is repeated little by little. Furthermore, in the stopped state, since the contact portion 31A of vibrating body of the piezoelectric actuator 31 is in a state of being fixed to the rotor 32, a resonant frequency of vibrating body during starting the drive is slightly different from a resonant frequency during steady vibration and it takes time when the rotor 32 reaches a rotating state from application start of the drive signal. Thus, it is considered that the efficiency of the low temperature mode is lower than that of the normal mode. Thus, if the rotor 32 is rotated at a predetermined rotation amount, power consumption of the normal mode is less than that of the low temperature mode.

As described above, in the embodiment, the control unit 44 can control driving of the pump 10 by the normal mode (first mode) and the low temperature mode (second mode). The control unit 44 performs the transporting method by the low temperature mode when the temperature measured by the temperature measuring unit (temperature sensor) 45 is lower than a predetermined value. In the low temperature mode, the control unit 44 transports the liquid by driving the pump 10 by the driving amount smaller than the driving amount (here, 20 counts) during the normal mode in the unit time shorter than the unit time (here, 0.5 seconds) during the normal mode. Thus, since the continuous driving time of the pump 10 can be reduced, it is possible to suppress the drop of the battery voltage during driving of the pump 10 even in the low temperature in which the current supply ability of the battery 42 is lowered (see Figs. 5A and 5B).

Furthermore, in the embodiment, the liquid transporting apparatus 1 has the boosting circuit (DC-DC converter) 43 that is the boosting circuit and supplies power to the pump 10 through the boosting circuit (DC-DC converter) 43. In such a configuration, if the battery voltage is the acceptable value or less, the voltage supplied from the boosting circuit (DC-DC converter) 43 does not maintain 3.3 V, but in the embodiment, since liquid transport is performed by the low temperature mode in the low temperature, it is possible to suppress that the battery voltage is the acceptable value or less. Thus, it is possible to maintain the output voltage of the boosting circuit (DC-DC converter) 43 to a predetermined voltage (3.3 V).

Furthermore, in the embodiment, the liquid transporting apparatus 1 includes the communication unit 46 as another power consumption element separate from the pump 10. The communication unit 46 is able to communicate independently from the control unit 44 and even though the command does not come out from the control unit 44, the communication unit 46 can perform communication. In this case, if the communication timing of the communication unit 46 overlaps with the driving timing of the pump 10, the voltage on the input side of the boosting circuit (DC-DC converter) 43 is greatly dropped and even if the battery voltage is slightly recovered after the communication completion, if driving of the pump 10 is continued, the battery voltage drops (see Figs. 3B and 3C). Then, as illustrated in Fig. 3C, in low temperature, the drop amount V2 of the battery voltage by driving of the pump 10 after the communication completion is greater than the recovery amount V1 of the battery voltage after the communication completion. In this case, there is a concern that the battery voltage is the acceptable value or less. However, in the embodiment, since the liquid transport is performed by the low temperature mode in low temperature, the driving timing of the pump 10 after the communication completion is decreased. Thus, the drop amount (corresponding to V2 of Fig. 3C) of the battery voltage after the communication completion is reduced. Thus, if it is designed such that the battery voltage is not the acceptable value or less when the communication timing of the communication unit 46 and the driving timing of the pump 10 are overlapped, it is possible to suppress that the battery voltage is the acceptable value or less by driving the pump 10 after the communication completion by performing the low temperature mode.

### Second Embodiment

In the first embodiment described above, the unit time during the normal mode is 0.5 seconds. However, the unit time during the normal mode may be other time. Furthermore, in the first embodiment described above, the low temperature mode is one type, but the low temperature mode may be two types or more.

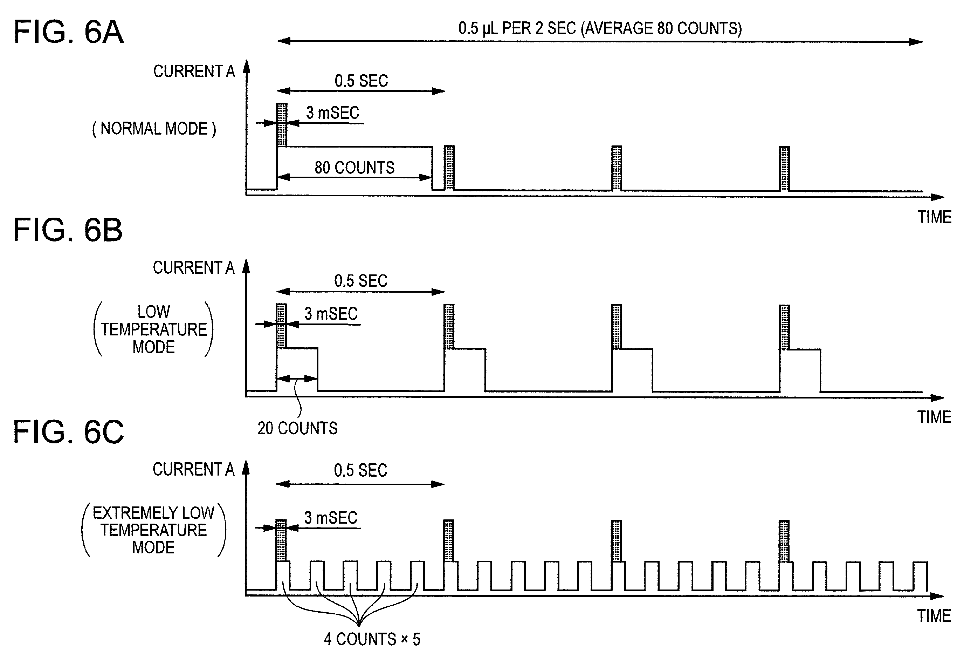

Figs. 6A to 6C are explanatory views of an output current of a boosting circuit (DC-DC converter) 43 of a second embodiment. Fig. 6A is an explanatory view of the output current of the boosting circuit (DC-DC converter) 43 during a normal mode. Fig. 6B is an explanatory view of the output current of the boosting circuit (DC-DC converter) 43 during a low temperature mode. Fig. 6C is an explanatory view of the output current of the boosting circuit (DC-DC converter) 43 during an extremely low temperature mode.

When a temperature measured by a temperature measuring unit (temperature sensor) 45 is lower than a first threshold value T1 (for example, 10°C), a control unit 44 transports a liquid by driving a pump 10 by a driving amount of 20 counts that is smaller than a driving amount (here, 80 counts) during the normal mode for every 0.5 seconds that is shorter than unit time (here, 2 seconds) during the normal mode (see Fig. 6B: low temperature mode).

Furthermore, when the temperature measured by the temperature measuring unit (temperature sensor) 45 is lower than a second threshold value T2 (for example, 5°C: T2<T1), the control unit 44 transports the liquid by driving the pump 10 by the driving amount of 4 counts that is smaller than the driving amount (here, 80 counts) during the normal mode for every 0.1 second that is shorter than the unit time (here, 2 seconds) during the normal mode (see Fig. 6C: extremely low temperature mode).

Also in the second embodiment, similar to the first embodiment, since a continuous driving time of the pump 10 can be reduced during the low temperature mode and during the extremely low temperature mode, it is possible to suppress drop of the battery voltage during driving of the pump 10 even in the low temperature in which the current supply ability of the battery 42 is lowered.

Furthermore, in the second embodiment, when the temperature measured by the temperature measuring unit (temperature sensor) 45 is lower than the second threshold value T2 that is a temperature lower than the first threshold value T1, a transporting method by the extremely low temperature mode is performed. In the extremely low temperature mode, the control unit 44 transports the liquid by driving the pump 10 by the driving amount that is smaller than the driving amount (here, 20 counts) during the low temperature mode in the unit time shorter than the unit time (here, 0.5 seconds) during the low temperature mode. Thus, it is possible to suppress drop of the battery voltage during driving of the pump 10 even in the extremely low temperature in which the current supply ability of the battery 42 is further lowered.

### Third Embodiment

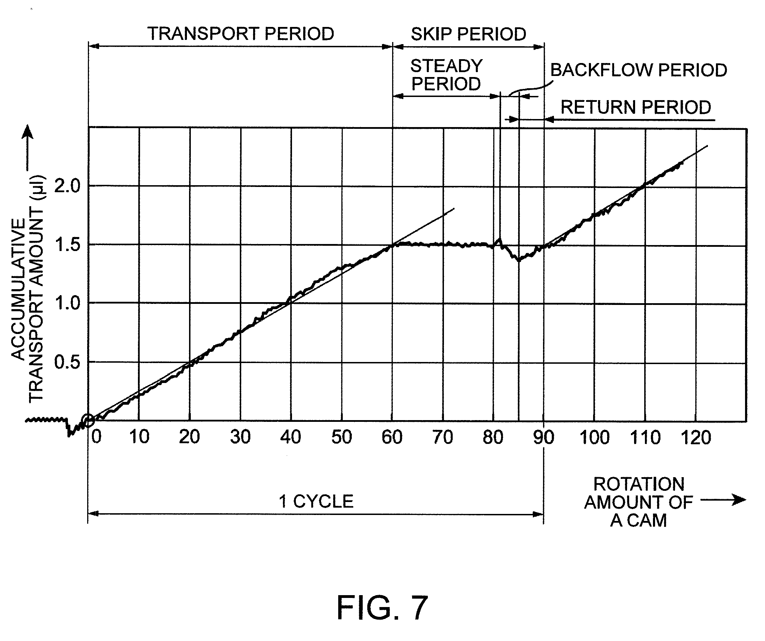

Fig. 7 is a graph of a relationship between a rotation amount of a cam 23 and an accumulative transport amount. In the graph, one position (reference position) of the cam 23 is 0 degree and accumulation of a transport amount of a liquid with respect to the rotation amount of the cam 23 from the reference position is calculated.

Here, the transport amount until the cam 23 rotates from 0 degree to 60 degrees (hereinafter, referred to as "transport period") is approximately proportional to a rotation angle. In the transport period, the liquid is in a state of being transported by closing a tube 21 by fingers sequentially from a first finger 22-1.

The accumulative transport amount is not changed when the cam 23 rotates from 60 degrees to 80 degrees (hereinafter, referred to as "steady period") . In the steady period, a seventh finger 22-7 (finger 22 on the most downstream side of a plurality of fingers 22) is in a state of continuously closing the tube 21.

When the cam 23 rotates from 80 degrees to 85 degrees (hereinafter, referred to as "backflow period"), the accumulative transport amount is reduced. That is, in the backflow period, the liquid flows back through the tube 21.

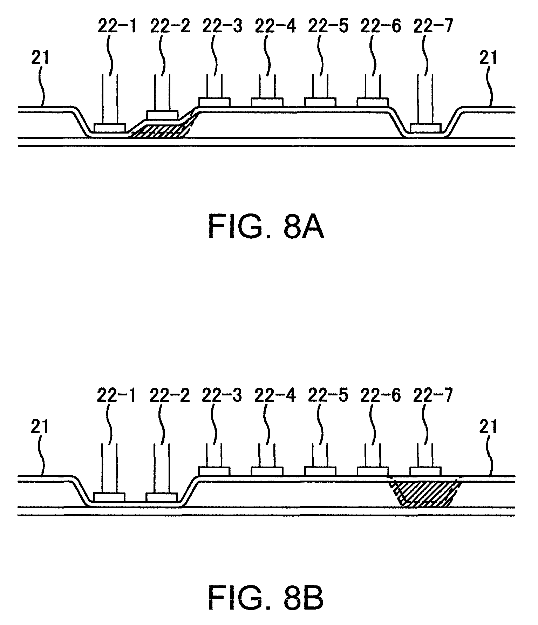

Figs. 8A and 8B are explanatory views of backflow of the liquid in the backflow period. The tube 21 is originally disposed in an arc shape, but here, for the sake of convenience, the tube 21 is disposed linearly.

The state of the tube 21 is moved from a state (see Fig. 8A) in which the seventh finger 22-7 closes the tube 21 to a state (see Fig. 8B) in which the tube 21 is opened by rotating the cam 23. In this case, the liquid flows back by a capacity of the capacity indicated by the hatched portion of Fig. 8B (more precisely, a capacity obtained by subtracting a capacity of a hatched portion of Fig. 8A from a capacity of a hatched portion of Fig. 8B).

Moreover, in a period (hereinafter, referred to as "return period") in which the cam 23 rotates from 85 degrees to 90 degrees, the liquid of an amount in compliance with the backflow is transported. Moreover, 0 degree that is the reference position is a position of the cam 23 after the return period. If the rotation of the cam 23 of 90 degrees is 1 cycle and a position in which the cam 23 rotates 90 degrees from the reference position is a new reference position of the next cycle, the transport period, the steady period, the backflow period, and the return period are present in each cycle. If the cam 23 is rotated 1 cycle, the liquid is transported in approximately equal amount (substantially 1.5 µL) regardless of a rotation start position of the cam 23. Since four protrusion units 23A are provided in the cam 23, the rotation of the cam 23 of 90 degrees is 1 cycle.

As described above, when rotating the cam 23, there are cases in which the liquid of the amount corresponding to the rotation amount of the cam 23 is transported, the liquid is not transported, and the liquid flows back. As a result, as illustrated in Fig. 7, the transport amount of the liquid with respect to the rotation amount of the cam 23 is different according to the rotation position of the cam 23. For example, even if the liquid is transported by rotating the cam 23 by 45 degrees, the transport amount (substantially 1.2 µL) when rotating the cam 23 from 0 degree that is the reference position to 45 degrees and the transport amount (substantially 0.3 µL) when rotating the cam 23 from 45 degrees to 90 degrees are different.

Then, the control unit 44 transports the liquid by rotating the rotor 32 by predetermined angles as usual in the transport period (period when the cam 23 rotates from 0 degree that is the reference position to 60 degrees). However, when the rotation position of the cam 23 reaches 60 degrees after the transport, the control unit 44 rotates the rotor 32 until it reaches the next transport period. That is, when the rotation position of the cam 23 reaches 60 degrees, the control unit 44 continuously drives the cam 23 such the cam 23 skips until that the rotation position of the cam 23 becomes 90 degrees (reference position of the next cycle). Thus, the control unit 44 can drive the cam 23 so as to skip the steady period, the backflow period, and the return period. As described above, driving the cam 23 to skip the steady period, the backflow period, and the return period is referred to as "skip drive". Since the transport amount of the liquid per time can be constant by performing the skip drive, it is possible to transport the liquid with high accuracy.

On the other hand, in the skip drive, since the cam 23 is required to be continuously driven such that the rotation position of the cam 23 is changed from 60 degrees to 90 degrees, a high load is applied to the battery 42 having low current supply ability. Furthermore, in the skip drive, the continuous driving time of the pump 10 is increased. Thus, if the skip drive is performed in the low temperature, the drop of the battery voltage is expected to be increased.

Thus, in the third embodiment, the control unit 44 performs the skip drive only when performing the normal mode and does not perform the skip drive when the temperature measured by the temperature measuring unit (temperature sensor) 45 is lower than a predetermined value and the low temperature mode is performed. Thus, it is possible to transport the liquid with high accuracy during the normal mode and it is possible to suppress the drop of the battery voltage during the low temperature mode.

### Fourth Embodiment

As described above, the current supply ability of the battery 42 is lowered during the low temperature. Then, in a fourth embodiment, the battery housing unit 41 housing the battery 42 has a heat insulating material for insulating the battery 42. In other words, the battery 42 is housed within the heat insulating material of the battery housing unit 41 and is insulated from the outside by the heat insulating material. Thus, even if an outside environment of the liquid transporting apparatus 1 is a low temperature, the temperature of the battery 42 is unlikely to be the low temperature and the battery 42 is likely to be kept warm by heat generated during use of the battery 42. Thus, it is possible to suppress the temperature drop of the battery 42 and to maintain the current supply ability of the battery 42.

As described above, if the battery 42 is housed in the heat insulating material, it is preferable that the temperature measuring unit (temperature sensor) 45 is also housed in the battery housing unit together with the battery 42. Thus, since the temperature measuring unit (temperature sensor) 45 can measure the temperature of the battery 42 with high accuracy, the control unit 44 can appropriately switch the normal mode and the low temperature mode according to the current supply ability of the battery 42.

### Fifth Embodiment

In the embodiments described above, the liquid transporting apparatus 1 includes one boosting circuit (DC-DC converter) 43 and power is supplied from the common boosting circuit (DC-DC converter) 43 to the pump 10 and the communication unit 46 (and the control unit 44). However, the liquid transporting apparatus 1 is not limited to the configuration.

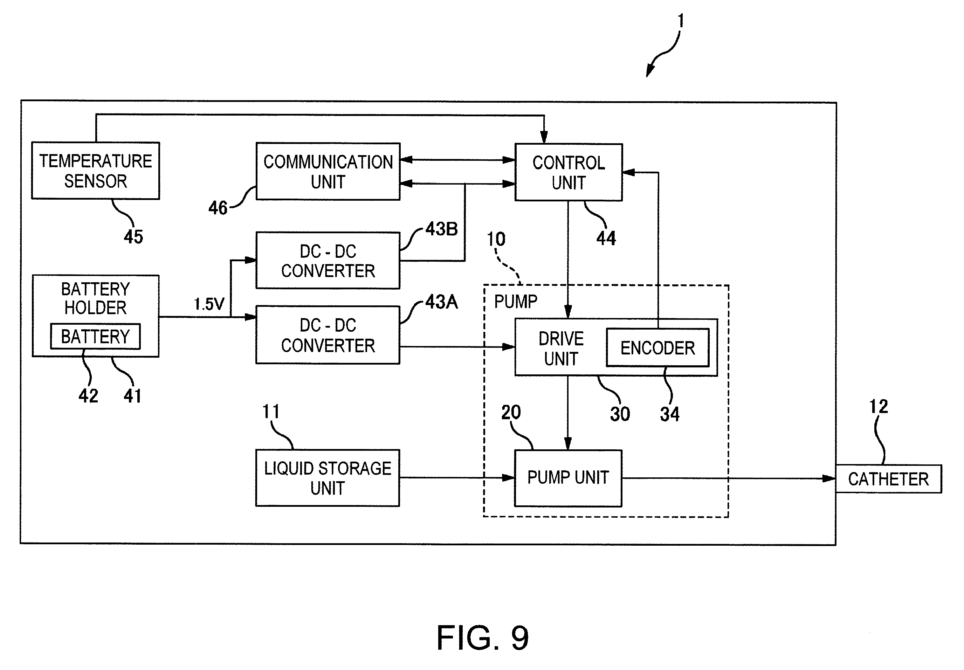

Fig. 9 is a block diagram of a liquid transporting apparatus 1 of a fifth embodiment. In the fifth embodiment, a liquid transporting apparatus 1 includes two boosting circuits (DC-DC converter) 43 (43A and 43B). Both the boosting circuits (DC-DC converter) 43 (43A and 43B) boost a power supply voltage (battery voltage) of 1.5 V. One boosting circuit (DC-DC converter) 43A supplies power to a pump 10 and the other boosting circuit (DC-DC converter) 43B supplies power to the communication unit 46 (and the control unit 44). Moreover, output voltages of two boosting circuits (DC-DC converter) 43 are different from each other.

Also in the fifth embodiment, it is preferable that the control unit 44 performs the transporting method by the low temperature mode when the temperature measured by the temperature measuring unit (temperature sensor) 45 is lower than a predetermined value. Thus, also during the low temperature in which a current supply ability of the battery 42 is lowered, it is possible to suppress drop of the battery voltage during driving of the pump 10.

Furthermore, also in the fifth embodiment, if the communication timing of the communication unit 46 overlaps with the driving timing of the pump 10, a voltage (the battery voltage) on an input side of the boosting circuits (DC-DC converter) 43 (43A and 43B) is greatly dropped. Furthermore, if driving of the pump 10 is continued even if the battery voltage is slightly recovered after the communication completion, the battery voltage is dropped (see Figs. 3B and 3C). Then, also in the fifth embodiment, during the low temperature, similar to a case of Fig. 3C, the drop amount V2 of the battery voltage by driving of the pump 10 after the communication completion is likely to be greater than the recovery amount V1 of the battery voltage after the communication completion and there is a concern that the battery voltage is the acceptable value or less. However, also in fifth embodiment, since the liquid transport is performed by the low temperature mode in the low temperature, the driving timing of the pump 10 after the communication completion is reduced. Thus, the drop amount (corresponding to V2 of Fig. 3C) of the battery voltage after the communication completion is reduced. Thus, also in the fifth embodiment, if it is designed such that the battery voltage is not the acceptable value or less when the communication timing of the communication unit 46 and the driving timing of the pump 10 are overlapped, it is possible to suppress that the battery voltage is the acceptable value or less by driving the pump 10 after the communication completion by performing the low temperature mode.

### Others

The embodiments described above are intended to facilitate the understanding of the invention and are not intended to be construed as limiting the invention. The invention can be changed, improved, and is of course to include equivalents thereof without departing from the spirit thereof.

### Liquid transporting apparatus 1

The liquid transporting apparatus 1 described above is a so-called insulin pump. However, the liquid transporting apparatus 1 is not limited to the insulin pump. Furthermore, the liquid transporting apparatus 1 may not be an apparatus for injecting a liquid into a living body or may be an apparatus for transporting the liquid.

### Pump 10

The pump 10 described above transports the liquid by squeezing the tube 21 by sequentially pressing the fingers 22 by the cam 23. However, the pump 10 is not limited to the device for transporting the liquid by squeezing the tube 21 and may be a device for transporting the liquid by another driving system.

## Claims

1. A liquid transporting apparatus transporting a liquid, comprising:
a pump unit that transports a liquid;
a drive unit that drives the pump unit by being supplied power from a battery;
a temperature measuring unit that measures a temperature; and
a control unit that controls the drive unit by a control of a first mode or a control of a second mode based on the temperature,
wherein the drive unit drives the pump unit by a predetermined driving amount per unit time as the control of the first mode, and
wherein the drive unit drives the pump unit by a driving amount that is less than the predetermined driving amount per unit time shorter than the unit time as the control of the second mode if the temperature is lower than a predetermined value.

2. The liquid transporting apparatus according to Claim 1, further comprising:
a boosting circuit that boosts a voltage of power supplied to the drive unit.

3. The liquid transporting apparatus according to Claim 1 or 2,
wherein the pump unit includes a tube, a plurality of fingers pressing the tube, a cam pressing the fingers,
wherein in the control of the first mode, a skip drive is performed to skip a period in which a finger on the most downstream side in a transport direction of a liquid closes the tube and the liquid is not transported, and a period in which the finger on the most downstream side is opened and the liquid flows back, and
wherein in the control of the second mode, the skip drive is not performed.

4. The liquid transporting apparatus according to any one of the preceding claims,
wherein a battery housing unit storing the battery includes a heat insulating material for insulating the battery.

5. The liquid transporting apparatus according to Claim 4,
wherein the temperature measuring unit is housed in the battery housing unit.

6. A liquid transporting method for transporting a liquid, the method comprising:
driving a pump unit by a drive unit by power supplied from a battery;
performing a control to drive the drive unit by a control unit by a predetermined driving amount per unit time as a control of a first mode; and
performing a control to drive the drive unit by the control unit by a driving amount that is less than the predetermined driving amount per unit time shorter than the unit time as a control of a second mode if the temperature measured by a temperature measuring unit is lower than a predetermined value.
